# EUROPEAN PATENT APPLICATION

(11) **EP 4 023 208 A1**
(43) Date of publication of application: **06.07.2022**
(21) Application number: 21169979.8
(22) Date of filing: 22.04.2021
(51) Int. Cl.: A61K 8/64, A61Q 17/00, A61Q 19/08

(54) **TOPICAL COMPOSITIONS FOR SKIN COMPRISING A CELL PENETRATING PEPTIDE AND METHODS OF USING THE SAME**

(30) Priority: 31.12.2020 TW 109147082
(71) Applicant: ImDerma Laboratories Co., Ltd., Hsinchu City 300 (TW)
(72) Inventor: Lai, Huey-Min, 300 Hsinchu City (TW); Huang, Yu-Lin, 103 Taipei City (TW)
(74) Representative: Lang, Christian

(57) **Abstract**

Topical compositions for skin include an active substance and a cell penetrating peptide consisting of following sequence: NYBX₁BX₂BNQX₃. The cell penetrating peptide is not only capable of delivering the active substance into skin cells effectively but also promoting the activity of the active substance.

## Description

### BACKGROUND OF THE INVENTION

### FIELD OF THE INVENTION

The present application relates to the skincare field, particularly the technique based on cell penetrating peptide for skincare.

### DESCRIPTION OF THE PRIOR ART

Air pollution or ultraviolet light may cause excessive peroxidation of skin and generations of intracellular free radicals and reactive oxygen species (ROS). ROS consists of minuscule particles including superoxide anions, hydrogen peroxide, hydroxyl radicals, etc. As highly reactive chemical molecules, excessive ROS is harmful to cell and gene structures and causes problems including inflammation and ageing. Because skin ageing and lots of diseases are related to ROS in cells, there have been many skin health products which emphasize the effects of anti-ageing and skincare by anti-oxidation such as eliminations of intracellular free radicals and ROS. The active substances for anti-oxidation used frequently are catechin, vitamin B3, vitamin C, retinoic acid, anti-inflammatory flavonoid or polyphenols. However, these active substances or other antioxidants well known to the public, each of which are difficultly absorbed by skin cells due to compound structures or skin barriers such as cuticles, are only modestly effective in eliminating intracellular ROS and free radicals or exerting other critically physiological functions.

The skin tissue includes cuticle, epidermis, dermis and subcutaneous connective tissue. Except for the cuticle which is a dead cell tissue, epidermis and dermis under the cuticle are made up of cells having activities and exerting some specific functions. According to the mainstream opinion of the percutaneous absorption theory, active substances which feature the molecular weight less than 500 Da, the structure with a specific ratio of lipophile, and peptides without a central polar or molecules with low polarity in the center have higher abilities to penetrate skin tissue and be absorbed by skin cells [1]. For most actives substances, penetrating the skin barriers is difficult, and it is also difficult for them to pass across cell membranes and enter the cells to exhibit their activities.

The study of the passage of drugs and actives substances through the skin has been valued recently because of market demand and prosperity in the skincare as well as the aesthetic medicine industry. There have been lots of enhancers or physical methods (for example, microneedle) developed for enhancing percutaneous absorption. However, besides inconvenience and high cost, those enhancers or physical methods may also damage the skin barrier and cause skin irritation or allergy [2,3]. Accordingly, new materials and methods for enhancing percutaneous absorption without damaging the skin barrier and causing skin irritation or allergy may be desirable.

Cell penetrating peptides (CPPs) are peptides that can deliver active substances such as compounds, proteins, peptides and nano-particles into cells. Most cell penetrating peptides contain an abundance of positively charged amino acids and can be internalized by cells after interacting with negatively-charged glycosaminoglycans (GAGs) and aggregating on the cell membrane surface [4]. For example, Tat (49-57) (RKKRRQRRR) from the tat protein transduction domain (PTD) of the human immunodeficiency virus type 1 (HIV-1) is able to penetrate cell membranes and deliver therapeutic proteins into mammalian cells after being modified [5]. However, cell penetrating peptides do not exhibit therapeutic ability such as elimination of reactive oxygen species. Besides, it normally needs to conjugate active substances to the cell penetrating peptides while uses of the cell penetrating peptides.

### Reference:

[1] Pickart, L.; Thaler, M.M. Tripeptide in human serum which prolongs survival of normal liver cells and stimulates growth in neoplastic liver. Nat. New Biol. 1973, 243, 85-87.
[2] Lopes, L.B.; Brophy, C.M.; Furnish, E.; Flynn, C.R.; Sparks, O.; Komalavilas, P.; Joshi, L.; Panitch, A.; Bentley, M.V. Comparative study of the skin penetration of protein transduction domains and a conjugated peptide. Pharm. Res. 2005, 22, 750-757.
[3] Degim, I.T. New tools and approaches for predicting skin permeability. Drug Discov. Today 2006, 11, 517-523.
[4] Chen, K.W.; Fuchs, G.; Sonneck, K.; Gieras, A.; Swoboda, I.; Douladiris, N.; Linhart, B.; Jankovic, M.; Pavkov, T.; Keller, W.; et al. Reduction of the in vivo allergenicity of Der p 2, the major house-dust mite allergen, by genetic engineering. Mol. Immunol. 2008, 45, 2486-2498.
[5] Park, J.; Ryu, J.; Kim, K.A.; Lee, H.J.; Bahn, J.H.; Han, K.; Choi, E.Y.; Lee, K.S.; Kwon, H.Y; Choi, S.Y. Mutational analysis of a human immunodeficiency virus type 1 Tat protein transduction domain which is required for delivery of an exogenous protein into mammalian cells. J. Gen. Virol. 2002, 83, 1173-1181.
[6] Gokce, E. H., Korkmaz, E., Dellera, E., Sandri, G., Bonferoni, M. C., & Ozer, O. (2012). Resveratrol-loaded solid lipid nanoparticles versus nanostructured lipid carriers: evaluation of antioxidant potential for dermal applications. Int J Nanomedicine, 7, 1841-1850.
[7] Aguirre, L., Fernandez-Quintela, A., Arias, N., & Portillo, M. P. (2014). Resveratrol: anti-obesity mechanisms of action. Molecules, 19(11), 18632-18655.
[8] Farris, P., Krutmann, J., Li, Y. H., McDaniel, D., & Krol, Y. (2013). Resveratrol: a unique antioxidant offering a multi-mechanisticapproach for treating aging skin. J Drugs Dermatol, 12(12), 1389-1394.
[9] Wenzel, E., & Somoza, V. (2005). Metabolism and bioavailability of trans-resveratrol. Mol Nutr Food Res, 49(5), 472-481.

### SUMMARY OF THE INVENTION

The present invention provides a cell penetrating peptide which can be combined used with active substances and rapidly enhancing the efficiencies of the activities of the active substance in skin tissues or cells. The cell penetrating peptide can be used as a penetration enhancer to deliver active substances into skin tissues/cells through not only being conjugated with the active substances but also being simply mixed with the active substances. The original functions of the active substances can be performed continuously; besides, the activities of the active substances become more efficient in the cells due to the cell penetrating peptide.

Accordingly, the present invention offers a topical composition for skin which comprises a cell penetrating peptide and an active substance. In the topical composition for skin, the cell penetrating peptide consisting of following sequence: NYBX₁BX₂BNQX₃, wherein N represents asparagine, Y represents tyrosine, B represents arginine or lysine, X1 represents tryptophan, alanine, phenoalanine or tyrosine, X₂ represents cysteine, Q represents glunine, X₃ represents asparagine or none.

To this end, the active substance comprises small molecule compounds, nucleic acids, nano-particles, polysaccharides, peptides or proteins.

To this end, the active substance is ethylbisiminomethylguaiacol manganese chloride, EUK-134^{™}, which is characteristic of the functional property of superoxide dismutase.

To this end, the active substance comprises cytokines.

To this end, the cytokines are transforming growth factor beta, TGF-β.

To this end, the active substance comprises phenolics.

To this end, the phenolics comprise flavonoids or stilbenes

To this end, the flavonoids comprise flavanole, flavanonole, chalkone, anthocy-anidine, aurone, flavone, flavanone or isoflavone.

To this end, the flavanole comprises catechin.

To this end, the catechin comprises epicatechin (EC), epigallocatechin (EGC), epicatechin gallate (ECG) or epigallocatechin gallate (EGCG).

To this end, the stilbenes comprise resveratrol.

To this end, the topical composition for skin is a topical liniment, a topical medicine, a topical skincare product or a cosmetic.

To this end, the topical skincare product is a toner, an emulsion, a factice, a cream, a moisturizer, a lip balm, an essence mask or a facial cleanser.

The present invention further offers an application of a cell penetrating peptide for skincare. In the application of a cell penetrating peptide for skincare, the cell penetrating peptide consisting of the following sequence: NYBX₁BX₂BNQX₃, wherein N represents asparagine, Y represents tyrosine, B represents arginine or lysine, X₁ represents tryptophan, alanine, phenoalanine or tyrosine, X₂ represents cysteine, Q represents glunine, X₃ represents asparagine or none.

To this end, the cell penetrating peptide is used in delivering an active substance into skin cells.

To this end, the active substance comprises an antioxidant or a substance facilitating secretions of extracellular matrices.

To this end, the antioxidant comprises polyphenols.

To this end, the polyphenols comprise tea polyphenols or resveratrol.

To this end, the antioxidant is ethylbisiminomethylguaiacol manganese chloride, EUK-134^{™}.

To this end, the substance facilitating secretions of extracellular matrices comprises transforming growth factor beta, TGF-β.

To this end, the extracellular matrices are collagens.

The present invention offers an application of a cell penetrating peptide for preparation of a medicinal composition for skin. In the application of a cell penetrating peptide, the cell penetrating peptide consisting of following sequence: NYBX₁BX₂BNQX₃, wherein N represents asparagine, Y represents tyrosine, B represents arginine or lysine, X₁ represents tryptophan, alanine, phenoalanine or tyrosine, X₂ represents cysteine, Q represents glunine, X₃ represents asparagine or none.

The cell penetrating peptide in the present invention is able to rapidly deliver active substances into skin cells through skin cell internalization or skin cell pinocytosis. The topical composition provided by the present invention has many advantages which include having the ability to pass through the cuticle layer of skin, entering cells and exhibiting functions thereof rapidly, and delivering biological cargo with safety. Accordingly, for the cosmetic industry, the cell penetrating peptide in the present invention is an effective and safe material for antipollution, anti-oxidation, anti-inflammation and ageing resistance. Moreover, it is the first innovative business application of the cell penetrating peptide and its family peptide in the skincare field.

The cell penetrating peptide itself is non-irritating, non-allergenic, non-damaging to the skin barrier, and has anti-inflammatory potential. The cell penetrating peptide can be combined used with active substances such as hydrophilic micromolecule, oleophilic micromolecule, slightly large peptide, protein and nano-particle. The active substances can be delivered into skin tissues or cells by the cell penetrating peptide; moreover, the activities of the active substances can be performed continuously and more efficiently. The composition comprising the cell penetrating peptide and an active substance can be used for external medicine, skin care, cosmetic care products, cosmeceuticals and make a wide range of important applications.

According to Grand View Research report, "The global anti-pollution skincare products market size was estimated at USD 9.07 billion in 2018 and is likely to expand further at a CAGR of 4.2% from 2019 to 2025". The market for UV protection against free radicals and intracellular reactive oxygen species are also rising. The composition comprising the cell penetrating peptide and an active substance (for example, tea polyphenols, resveratrol, SOD or other active molecules) will provide an excellent solution to market demand.

In summary, the present invention provides an application of a cell penetrating peptide for skincare or preparation of a medicinal composition for skin and a topical composition for skin comprising the cell penetrating peptide. The cell penetrating peptide functioning as a good carrier of an active substance facilitates cellular entry of an active substance into skin cell; wherein the active substance comprises peptide, protein, growth factor, nano-particle, polysaccharide, DNA, RNA or other active substances with pharmacological activities. The cell penetrating peptide is non-toxic and it can be combined used with an active substance to enhance the effect of the active substance. The cell penetrating peptide is applicable to developments and preparations of new skincare cosmetics, topical medicines for skin or other related products.

These features and advantages of the present invention will be fully understood and appreciated from the following detailed description of the accompanying Drawings.

### BRIEF DESCRIPTION OF THE DRAWINGS

FIG. 1 illustrates the effect of a cell penetrating peptide on the intracellular levels of reactive oxygen species (ROS) induced by hydrogen peroxide (H₂O₂);
FIG. 2 illustrates the effect of tea polyphenols on the intracellular levels of ROS induced by H₂O₂;
FIG. 3 illustrates the effect of combined use of a cell penetrating peptide and tea polyphenols on the intracellular levels of ROS induced by H₂O₂;
FIG. 4 illustrates the effect of EUK-134^{™} on the intracellular levels of ROS induced by H₂O₂;
FIG. 5 illustrates the effect of combined use of a cell penetrating peptide and EUK-134^{™} on the intracellular levels of ROS induced by H₂O₂;
FIG. 6 illustrates the effect of combined use of a cell penetrating peptide and resveratrol on the intracellular levels of ROS induced by H₂O₂; and
FIG. 7 illustrates the effect of combined use of a cell penetrating peptide and TGF-β on the expression of Type 1 pro-collagen.

### DETAILED DESCRIPTION OF THE PREFERRED EMBODIMENT

All technical and scientific terms used in the specification refer to meanings well known to persons with ordinary skills in the art unless otherwise stated.

The singular terms, "a", "an" and "the", used in this specification and the scope of the patent application may refer to more than one subject unless otherwise stated.

The term, "fold of control", used in the ordinate of the drawings in this specification refers to the relative value to the control (the value of the control group is specified as 1).

"Or", "and", and "as well as" used in this specification refer to "or/and" unless stated otherwise. In addition, the terms, "including" and "comprising", are open-ended connectives without restrictions. The preceding paragraph is a systematic reference only and should not be construed as a limitation on the subject of the invention.

The term, "combined use", or other approximate terms used in the specification refer to at least one selected medicine given to a patient and one course of treatment in which one or more medicines are administrated to a patent simultaneously.

A substance or a composition is compatible to other ingredients in a concoction and harmless to patients.

A carrier acceptable in medicines or cosmetics could include one or more reagents selected from the following group: solvent, emulsifier, suspending agent, decomposer, binding agent, excipient, stabilizing agent, chelating agent, diluent, gelling agent, preservative, lubricant, surfactant, and another similar or applicable carrier.

A cell penetrating peptide in the present invention is applicable to skincare or manufactured as a medicinal composition for skin; the dosage form for a cell penetrating peptide which is adjustable as required is not limited to but presented as a topical dosage form preferably.

All materials used in the present invention are available in the market unless otherwise stated.

The cell penetrating peptide in the present invention consisting of the following sequence: NYBX₁BX₂BNQX₃, wherein N represents asparagine, Y represents tyrosine, B represents arginine or lysine, X₁ represents tryptophan, alanine, phenoalanine or tyrosine, X₂ represents cysteine, Q represents glunine, X₃ represents asparagine or none; the cell penetrating peptide is selected from a peptide containing 10 residues which are the major GAG (glycosaminoglycan) binding motif of ECP (eosinophil cationic protein; Uniprot No.: P12724; Gene ID: 6037). As disclosed in TWI406946B, mutations and replacement with other residues at several positions of the peptide are tolerable and the mutated peptide is still capable of binding to cells proven by the assays of QuickChange site-directed mutagenesis technique and the enzyme-linked immunosorbent assay (ELISA).

The cell penetrating peptide in the present invention is produced by a traditional chemical synthesis method which includes, without limitation, liquid-phase synthesis or solid-phase synthesis. Moreover, the cell penetrating peptide is also produced by the recombinant DNA technology (in which nucleic acid molecules, carriers or host cells are recombined).

Both a cell penetrating peptide in the present invention and an active substance can be manufactured as a mixed combination, which includes or excludes surfactants or alcohols, and further a water-in-oil (W/O) emulsion or oil-in-water (O/W) emulsion; moreover, the mixed combination has the form of reversed micelle or liposome. The reversed micelle is made of a solid material. For example, solid materials The reversed micelle can be made through the following process: melting solid materials to at around 70 °C and stirring the solid materials for several minutes with heating; subsequently, adding an aqueous solution of the cell penetrating peptide and the active substance dropwise to the heated solid materials (hydrophobic phase); wherein the solid material includes, without limitation, isopropyl myristate, isopropanol, glyceryl monooleate, isopropyl stearate, isopropyl palmitate, butanediol, propyl glycol or 1-ethyl-3(3-dimethylaminopropyl)-carbodiimide (EDC). Alternatively, the cell penetrating peptide and the active substance can be coated by the ordinary methods of liposome preparation; wherein the liposome may include phospholipid or cholesterol. In the mixed combination, the concentration of the cell penetrating peptide ranges from 0.01 µM to 5µM and the concentration of the active substance relying on purposes range from 0.001 µM to 5µM in general.

For an optimal effect, the mixed combination comprises isopropyl myristate, isopropanol, glyceryl monooleate and water; wherein isopropyl myristate, isopropanol, glyceryl monooleate and water in the mixed combination range from 80% to 92%, from 2% to 10%, from 4% to 8%, and from 0.5% to 4% by weight, respectively.

Tea polyphenols used in the present invention is available from the market or extracted with a general method known to persons skilled in the art. For example, tea polyphenols can be prepared in steps as follows: extracting and collecting tea polyphenols extracts from tea leaves and twigs with hot water; removing lipids, proteins, some polysaccharose and alkaloids from the tea polyphenols extracts by the membrane separation method; further removing caffeine in the tea polyphenols by using ethanol as a solvent with a specific resin; after removing the ethanol, the tea polyphenols is obtained. In embodiments of the present invention, the concentration of epigallocatechin gallate (EGCG) included in tea polyphenols is specified as but not limited to 7.25%.

The technical features for novelty and specific features in the present invention are disclosed in appended claims; the detailed technical features in the specification will be elaborated and comprehended by preferable embodiments and accompanying drawings for the inventive principle.

The present invention is illustrated but not limited to the following embodiments.

### Embodiment 1: preparations of a cell penetrating peptide and the mixed combination thereof

The cell penetrating peptide with the sequence of Asn-Tyr-Arg-Trp-Arg-Cys-Lys-Asn-Gln-Asn (NYRWRCKNQN; SEQ ID No: 1; containing 10 amino acids with molecular weight about 1381.54) was synthesized from C-terminal to N-terminal by solid-phase synthesis. After deprotecting of protecting groups and dialysis, the cell penetrating peptide was purified with the reversed phase high performance liquid chromatography (RP-HPLC); subsequently, the purified cell penetrating peptide (hereinafter referred to as peptide X) was lyophilized and stored at -20°C until use. Having tested cell penetrating peptides, the patent applicant found no significant difference in the results from Embodiment 2 to Embodiment 6 with the cell penetrating peptide (SEQ ID No: 1) replaced by another cell penetrating peptide with the sequence of Asn-Tyr-Arg-Tyr-Arg-Cys-Lys-Asn-Gln-Asn (NYRYRCKNQN; SEQ ID No: 2; containing 10 amino acids with molecular weight about 1358.50). Moreover, the cell penetrating peptide (sequence: NYBX₁BX₂BNQX₃; N: asparagine; Y: tyrosine; B: arginine or lysine; X₁: tryptophan, alanine, phenoalanine or tyrosine; X₂: cysteine; Q: glunine; X₃: asparagine or none) has no significant effect on the results from Embodiment 2 to Embodiment 5.

### Embodiment 2: feasibility of delivering tea polyphenols using peptide X

Tea polyphenols which is a generic term for polyphenols in tea have the effects of eliminating free radicals and anti-oxidation. According to researches for anti-oxidation, tea polyphenols present better anti-oxidation than other polyphenols. The four types of tea polyphenols, epicatechin (EC), epigallocatechin (EGC), epicatechin-3-gallate (EGC) and epigallocatechin-3-gallate (EGCG) are studied and explored frequently. In which, EGCG is most prevalent and has the greatest biological activity. Although it has been proved that the tea polyphenols have anti-oxidation activities, the effects of the anti-oxidation activities are limited because it is difficult for tea polyphenols to enter cells due to their flat structures.

In order to confirm if the peptide X is capable of delivering tea polyphenols into skin cells and its effect on the anti-oxidation activities of tea polyphenols, 100µL of HaCaT cells, human keratinocyte line, were pre-seeded in a 96-well plate at the density of 2x10⁶/mL one day before treatment. Following the removal of DMEM medium in the 96-well plate, 100 uL of 1µM peptide X and 100 uL of tea polyphenols (0.01%, 0.05% or 0.1%), were added in the 96-well plate. After 2, 4, 6, 16 or 24 hours of reaction, the peptide X and tea polyphenols were removed and then DMEM medium with 1.2 mM H₂O₂ was added for 2 hours of reaction in dark to induce intracellular ROS in the HaCaT cells. After the above reaction, DMEM medium with 1.2 mM H₂O₂ was removed from the 96-well plate and 100 µL of 10µM DCFDA (2',7'-dichlorofluorescin diacetate) was added for half an hour of reaction to measure the levels of intracellular ROS in HaCaT cells using fluorescence assay. Following the removal of DCFDA, the treated cells were washed with 200 µL of PBS. Subsequently, after PBS was removed, 100 µL of transparent DMEM medium was added and absorbance at 488/520 nm was measured to determinate the levels of intracellular ROS. After the above measurement, the transparent DMEM medium was removed and then 100µL of 1mg/mL MTT was added for 2 hours of reaction; subsequently, the absorbance at 595 nm was measured for calculating the cell viability.

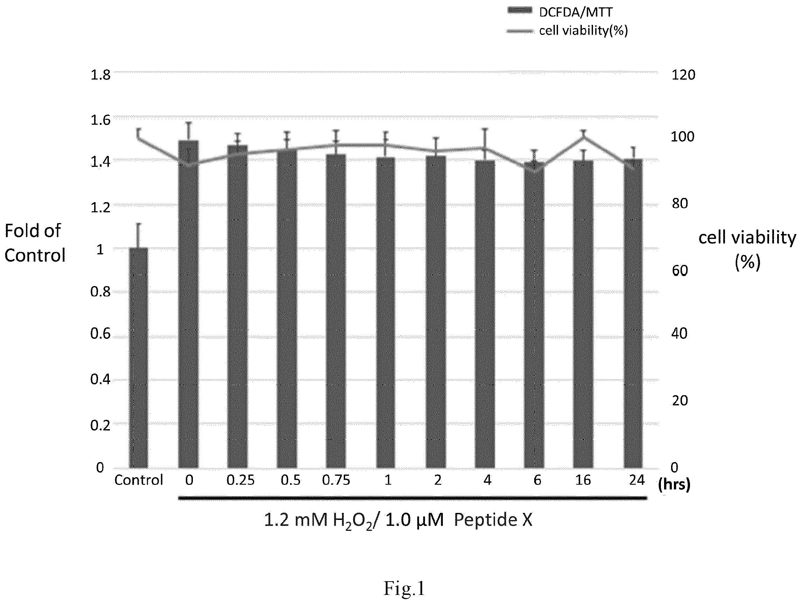

It can be seen from the results in FIG. 1 that treatment with 1.0 µM peptide X shows no cytotoxicity to HaCaT cells and not altered the levels of intracellular ROS induced by H₂O₂, that is, peptide X does not have the function of eliminating intracellular ROS.

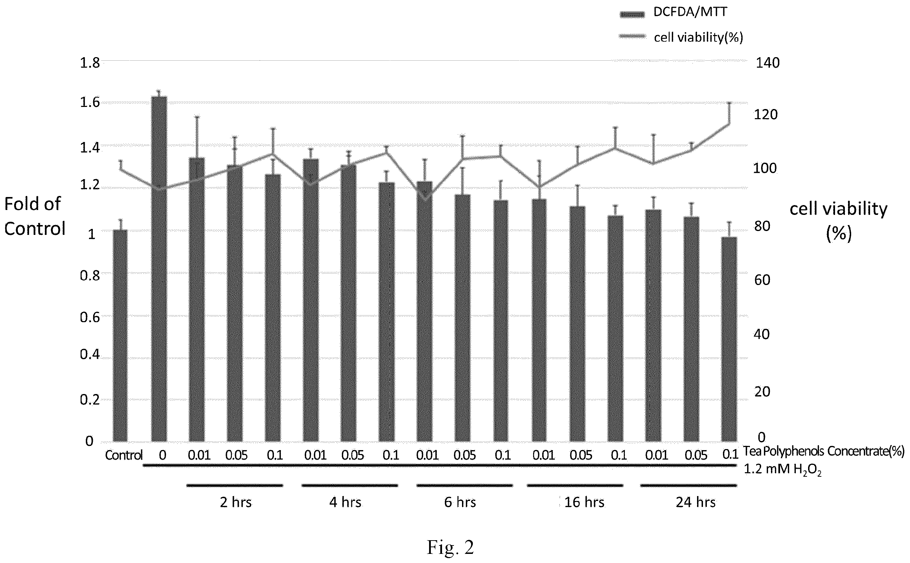

It can be seen from test results in FIG. 2 that the levels of intracellular ROS induced by H₂O₂ decrease with the increasing concentration of tea polyphenols (the group labeled with 0 hour in FIG. 2 was only treated with H₂O₂ but not treated with tea polyphenols) and as the cells were treated with tea polyphenols for longer (2 hours, 4 hours, 6 hours, 16 hours, 24 hours), the decrease in the levels of the intracellular ROS was increased gradually. Besides, cells need to be treated with 0.1% tea polyphenols for 24 hours so that the levels of the intracellular ROS can be reduced to the same levels of that of the control group. Moreover, according to the results of FIG. 2, tea polyphenols (0.01%, 0.05% or 0.1%) including 7.25% EGCG are non-toxic to HaCaT cells.

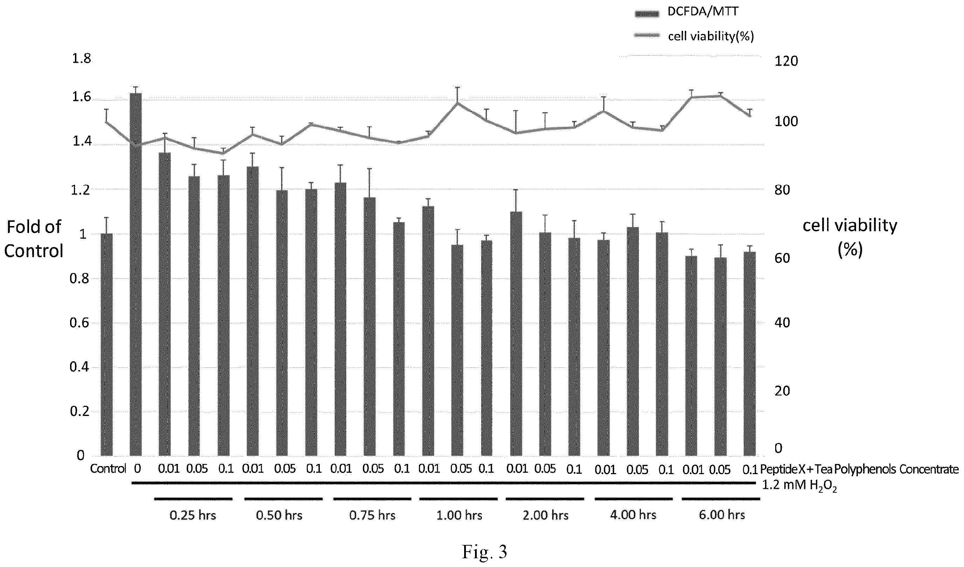

It can be seen from test results in FIG. 3 that co-treatment with 1.0 µM peptide X and tea polyphenols (0.01%, 0.05% or 0.1%) shows no cytotoxicity to HaCaT cells and the levels of the intracellular ROS significantly decrease after 15 minutes of treatment with peptide X and tea polyphenols. The levels of the intracellular ROS in cells which were treated with 0.01%, 0.05% and 0.1% tea polyphenols were decreased 64%, 74% and 92% after 45 minutes of treatment, respectively. Besides, the levels of the intracellular ROS in cells treated with 0.05% or 0.1% tea polyphenols were reduced to the same levels of that of the control group after one hour of treatment. The results demonstrate that peptide X meets the expectation that it is capable of facilitating cellular uptake of tea polyphenols and is capable of enhancing the efficiency of the anti-oxidation activities of tea polyphenols.

According to FIG. 1 to FIG. 3, peptide X facilitates the rapid cellular entries of tea polyphenols (contain 7.25% EGCG) which are difficult to pass through cell membranes due to their flat structures and the anti-oxidation activities of the water soluble polyphenols tea are still maintained. Moreover, it only needs 1.0 µM of peptide X for 24 times enhancement of the efficiency of anti-oxidation activities compared to that of tea polyphenols without peptide X at the same concentration.

### Embodiment 3: feasibility of delivering EUK-134^{™} using peptide X

As a mimic of superoxide dismutase (SOD), ethylbisiminomethylguaiacol manganese chloride (EUK-134^{™}), which is an artificially synthesized compound with the activity of SOD, is a compound material for skincare cosmetics sold by Lucas Meyer Cosmetics. Featuring the function of the activity of an antioxidant (the function of EUK-134^{™} registered in the International Nomenclature of Cosmetic Ingredients (INCI)), EUK-134^{™}, as a raw material for applications of skincare cosmetics, is applicable to different types of skincare cosmetics for anti-oxidation, ageing resistance and anti-inflammation.

In order to confirm if the peptide X is capable of delivering EUK-134^{™} into skin cells and its effect on the anti-oxidation activities of EUK-134^{™}, the method in Embodiment 2 is referred. EUK-134^{™} (0.12 µM, 0.23 µM or 2.35 µM) or a mixture of peptide X (1µM) and EUK-134^{™} (0.12 µM or 0.23 µM), were added in HaCaT cells for 8 hours of reaction. Following the removal of EUK-134^{™} and the mixture of peptide X and EUK-134^{™}, 1.2 mM H₂O₂ was added to induce intracellular ROS in the HaCaT cells and then the levels of intracellular ROS were detected using DCFDA. After the detection of the levels of intracellular ROS, the cell viability was confirmed using MTT assay.

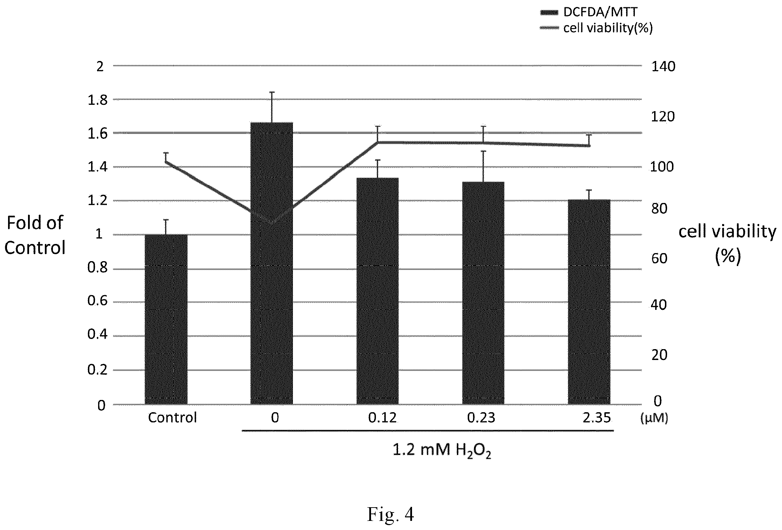

It can be seen from the results in FIG. 4 that treatment with 1.0 µM peptide X shows no cytotoxicity to HaCaT cells and in the group of treatment without peptide X, the level of intracellular ROS induced by H₂O₂ was slightly decreased with the increase of the treatment concentration of EUK-134^{™}. The level of intracellular ROS induced by H₂O₂ can be reduced by 50% using 0.12 µM EUK-134^{™} for 8 hours of treatment.

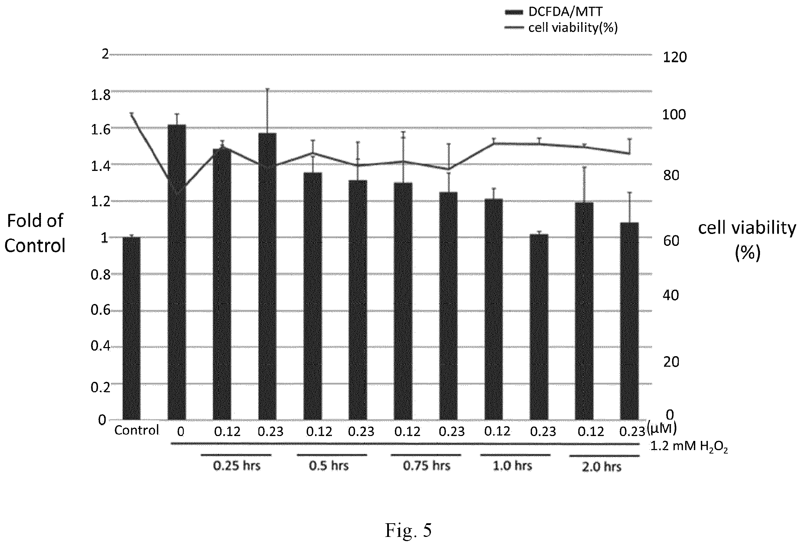

It can be seen from the results in FIG. 5 that treatment with the mixture of peptide X (1µM) and EUK-134^{™} (0.12 µM or 0.23 µM) shows no cytotoxicity to HaCaT cells according to the result of cell viability and the phenomenon of intracellular ROS inhibition was found after 45 minutes treatment of peptide X (1µM) and EUK-134^{™} (0.12 µM); the level of intracellular ROS can be reduced by 50%. The efficiency of co-treatment with peptide X (1µM) and EUK-134^{™} on intracellular ROS elimination is 10.67 times higher than that of treatment with EUK-134^{™}(0.12µM for 8 hours).

According to FIG. 4 and FIG. 5, peptide X is capable of delivering EUK-134^{™}, a compound with the activity of SOD, into skin cells rapidly. Co-treatment with 1µM peptide X and 0.23 µM EUK-134^{™} shows no cytotoxicity to HaCaT cells according to the result of cell viability. The level of intracellular ROS induced by 1.2 mM H₂O₂ can be reduced to the same level of that of control group by one hour co-treatment with 1µM peptide X and 0.23 µM EUK-134^{™}; however, the level of intracellular ROS can only be reduced by 50% using 0.23 µM EUK-134^{™} (without peptide X) even for 8 hours of treatment. In summary of the results, peptide X is capable of raising the efficiency of ROS elimination of EUK-134^{™} to 10.67 times. Peptide X can not only rapidly deliver EUK-134^{™} into epidermal cells, but also enhance the efficiency of EUK-134^{™} as a superoxide dismutase.

### Embodiment 4: feasibility of delivering resveratrol using peptide X

Resveratrol is a phenolic compound widely found in many plants such as grapes, peanuts, lilies, and pine trees. Resveratrol is a kind of phytoalexin. When plants are exposed to environmental pressure, fungi and bacterial infections, resveratrols will be produced to fight against the invasion of foreign objects. Many literatures have pointed out that resveratrol has good anti-oxidation, anti-photoaging, anti-inflammatory, anti-cell proliferation [6] and cardiovascular protective effects [7], and it also has inhibitory effects on melanin biosynthesis [8]. However, chemical stability, poor solubility and low bioavailability of resveratrol limit its development and application process [9].

The structural formula of resveratrol is shown as follows:

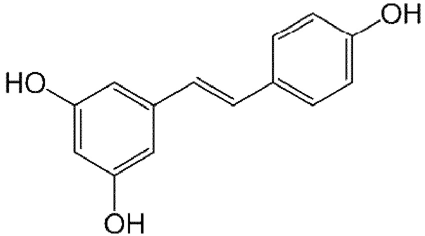

In order to confirm if the peptide X is capable of delivering resveratrol into skin cells, the method in Embodiment 2 was referred. Resveratrol (6.25 µM, 12.5 µM or 25 µM) or a mixture of peptide X (1µM) and resveratrol (6.25 µM, 12.5 µM or 25 µM), were added in HaCaT cells for different time of reaction. Following the removal of resveratrol and the mixture of peptide X and resveratrol, 1.2 mM H₂O₂ was added to induce intracellular ROS in the HaCaT cells and then the levels of intracellular ROS were detected using DCFDA. After the detection of the levels of intracellular ROS, the cell viability was confirmed using MTT assay.

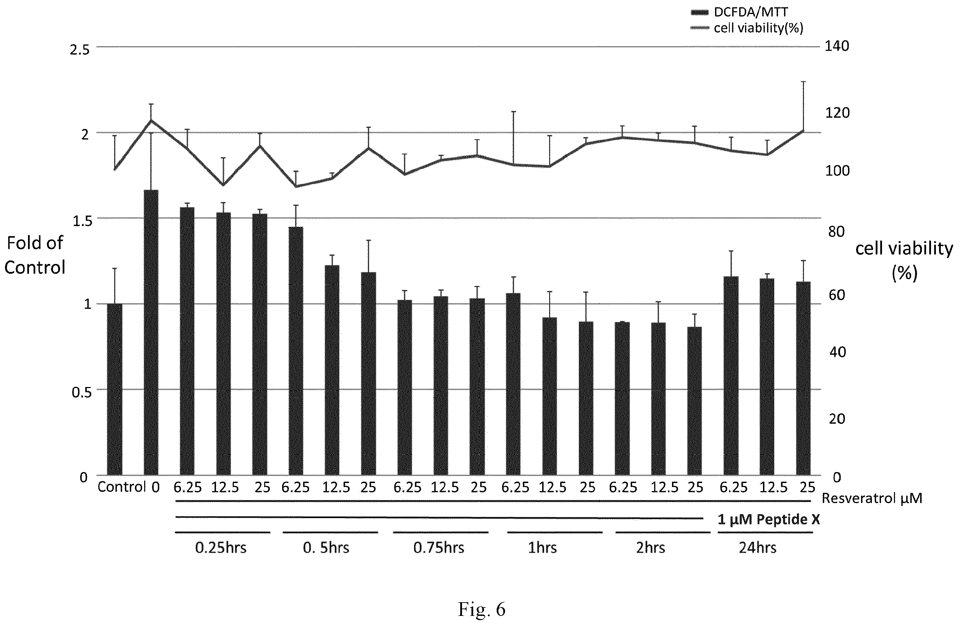

It can be seen from the results in FIG. 6 that the mixture of peptide X (1µM) and resveratrol (6.25 µM, 12.5 µM or 25 µM) shows no cytotoxicity to HaCaT cells and it can be seen from the groups of treatment without peptide X (the three groups in the right hand side of FIG. 6 which were only treated with resveratrol (6.25 µM, 12.5 µM or 25µM)) that the level of intracellular ROS induced by H₂O₂ was reduced by 75∼80% using resveratrol, although there is no significant difference among the groups treated with different concentrations of resveratrol. It should be noted that the efficiencies of intracellular ROS elimination are significantly enhanced in the groups treated with the mixture of peptide X (1µM) and resveratrol, compared to the groups treated without peptide X. Taking the group treated with the mixture of peptide X (1µM) and resveratrol (6.25 µM) as an example, it only needs 0.75 hours of reaction to reduce the intracellular ROS to the same level as that of control. The efficiency of the mixture comprising peptide X and resveratrol on intracellular ROS elimination is at least 30 times higher than that of resveratrol. It has also been observed from the results that peptide X promotes the effect of resveratrol on eliminating intracellular ROS. When the reaction time is short (for example, within 30 minutes), the concentration of resveratrol will affect the elimination effect, resveratrol with higher concentration is more effective in eliminating intracellular ROS. But if the reaction time is extended, the effect of the action time factor is obviously more important than the concentration of resveratrol for the effect of peptide X in promoting resveratrol to eliminate intracellular ROS.

Accordingly, peptide X can not only rapidly deliver resveratrol into skin cells, but also greatly enhance the effect of resveratrol on eliminating intracellular ROS.

### Embodiment 5: feasibility of delivering transforming growth factor beta (TGF-β) using peptide X

Transforming growth factor beta (TGF-β) can promote the downstream Smad2 (Mothers against decapentaplegic homolog 2) to induce the synthesis of type I pro-collagen, which leads to the increase of collagen in the dermis. In the field of skin care products, TGF-β is usually used to promote the proliferation of collagen to help maintain skin's elasticity.

In order to confirm if the peptide X is capable of delivering TGF-β into skin cells, Hs68 cells were seeded in a 6-well plate. Following the removal the medium in the 6-well plate, TGF-β (10 ng/mL), peptide X (1.0 µM) or a mixture of TGF-β (10 ng/mL) and peptide X (1.0 µM) were added in the 6-well plate. After 6, 12 or 24 hours of reaction, the level of type I pro-collagen in the cells was confirmed using ELISA kit with human pro-collagen 1 alpha 1 capture antibody and human pro-collagen 1 alpha 1 detector antibody.

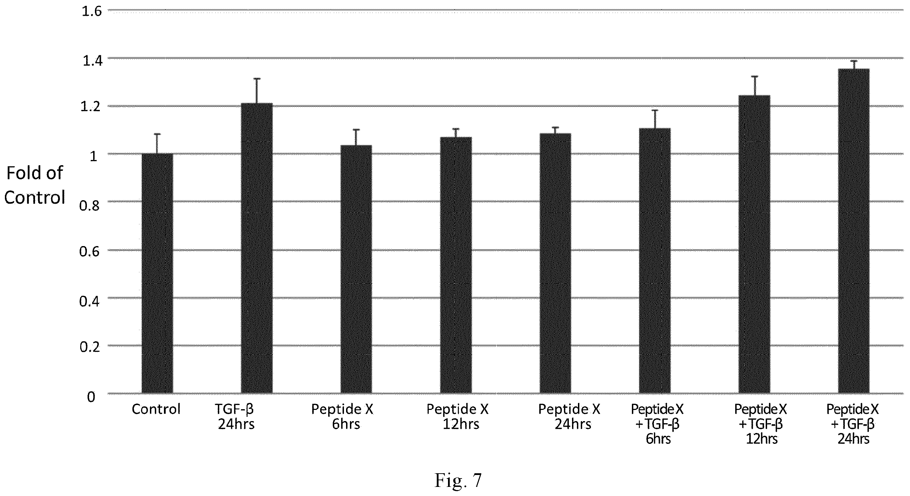

The result is shown in FIG. 7. Cells in all experimental groups grow normally. The expression of type I pro-collagen increases by 20% or so after the addition of TGF-β (10 ng/mL) but the expression of type I pro-collagen in the group with the addition of peptide X (1.0 µM) only increases slightly with extended reaction time. However, within the same reaction time (24 hours), the expression of type I pro-collagen in the group with an addition of the mixture of TGF-β (10 ng/mL) and peptide X (1.0 µM) is over 25% higher than that in the group with an addition of TGF-β (10 ng/mL) only. The test results demonstrate that co-treatment of peptide X and TGF-β further promote the formation of type I pro-collagen in human fibroblasts (Hs68 cells)

According to the results, it can be seen that the cell penetrating peptide of the present invention is not toxic to skin cells and can achieve the following effects:
(1) It can rapidly deliver tea polyphenols, which are originally difficult to enter cell membranes due to their good water solubility and structure into epidermal cells, and the delivered tea polyphenols can still function as water-soluble super antioxidants continuously and even more efficiently. Moreover, it only needs 1.0 µM of peptide X for enhancing efficiencies of tea polyphenols on intracellular ROS elimination by 24 times, compared to that of tea polyphenols without peptide X at the same concentration.
(2) It can rapidly deliver EUK-134^{™}, a compound with the function of SOD, into epidermal cells, and the delivered EUK-134^{™} can still function as SOD continuously and even more efficiently. Moreover, it only needs 1.0 µM of peptide X for enhancing the efficiency of EUK-134^{™} (0.23 µM) on intracellular ROS elimination by 10.67 times.
(3) It can rapidly deliver resveratrol into epidermal cells, and the delivered resveratrol can function as fat-soluble super antioxidants more efficiently. Moreover, it only needs 1.0 µM of peptide X for enhancing the efficiency of resveratrol (6.25 µM) on intracellular ROS elimination by 30 times.
(4) It can deliver growth factor proteins with a large molecular weight, for example, TGF-β, into epidermal cells, and the delivered TGF-β can perform its function, promoting formation of type I pro-collagen continuously and more quickly. The combined use of 1µM peptide X can increase the effect of TGF-β in promoting the formation of type 1 pro-collagen by approximately 25%, compared to the treatment without peptide X.

Accordingly, the cell penetrating peptide in the present invention can not only rapidly enter cells itself, but also has the function of delivering active substances into cells; the cell penetrating peptide can promote the speed and efficiency of the cell internalization of active substances; wherein the active substance includes, without limitation, hydrophilic small molecules, lipophilic small molecules, larger peptides, proteins, growth factors, nanoparticles, polysaccharides, DNA or RNA.

The results of the embodiment in the present invention can prove that the anti-oxidant function of the active substances delivered by the cell penetrating peptide can not only be performed, but also be greatly enhanced due to the rapid cell internalization or other interaction factors. Moreover, the cell penetrating peptide in the present invention is capable of delivering growth factors with lager molecular weights and enhancing the effect of growth factors on promoting the secretion of extracellular matrix by skin cells.

The descriptions presented in preferred embodiments of the specification are only examples clearly understood by a person with ordinary knowledge in the art; the embodiments can be implemented by the person with ordinary knowledge in the art through many modifications or changes of the embodiments without difference from technical characteristics of the present invention. Despite many modifications or changes based on embodiments of the specification, the present invention is still embodied. The scope of the present invention is defined as disclosed in appended claims in the specification and incorporates the above methods, architecture and other equivalent inventions

As presented in many effects hereinbefore, a cell penetrating peptide and an active substance for promoting the efficiency of the active substance and skincare in the specification meets novelty and non-obviousness for patentability.

Many changes and modifications in the above described embodiment of the invention can, of course, be carried out without departing from the scope thereof. Accordingly, to promote the progress in science and the useful arts, the invention is disclosed and is intended to be limited only by the scope of the appended claims.

## Claims

1. A topical composition for skin, comprising a cell penetrating peptide and an active substance, wherein the cell penetrating peptide consists of the following sequence: NYBX₁BX₂BNQX₃, wherein N represents asparagine, Y represents tyrosine, B represents arginine or lysine, X₁ represents tryptophan, alanine, phenoalanine or tyrosine, X₂ represents cysteine, Q represents glunine, and X₃ represents asparagine or none.

2. The topical composition for skin as claimed in claim 1, wherein the active substance comprises small molecule compounds, nucleic acids, nano-particles, polysaccharides, peptides or proteins.

3. The topical composition for skin as claimed in claim 1, wherein the active substance is ethylbisiminomethylguaiacol manganese chloride (EUK-134^{™}).

4. The topical composition for skin as claimed in claim 1, wherein the active substance comprises cytokines.

5. The topical composition for skin as claimed in claim 1, wherein the active substance comprises phenolics.

6. The topical composition for skin as claimed in claim 5, wherein the phenolics comprise flavonoids or stilbenes.

7. The topical composition for skin as claimed in claim 6, wherein the flavonoids comprise catechin and the stilbenes comprise resveratrol.

8. The topical composition for skin as claimed in claim 1, wherein the topical composition for skin is a topical liniment, a topical medicine, a topical skincare product or a cosmetic.

9. A method of a skin treatment comprising:
administering a topical composition comprising a cell penetrating peptide and an active substance to an individual in need thereof;
wherein the cell penetrating peptide consists of the following sequence:
NYBX₁BX₂BNQX₃, wherein N represents asparagine, Y represents tyrosine, B represents arginine or lysine, X₁ represents tryptophan, alanine, phenoalanine or tyrosine, X₂ represents cysteine, Q represents glunine, and X₃ represents asparagine or none.

10. The method as claimed in claim 9, wherein the cell penetrating peptide is used to deliver the active substance into skin cells.

11. The method as claimed in claim 10, wherein the active substance is an antioxidant or a substance facilitating secretions of extracellular matrices.

12. The method as claimed in claim 11, wherein the antioxidant comprises polyphenols.

13. The method as claimed in claim 11, wherein the antioxidant comprises tea polyphenols, resveratrol or ethylbisiminomethylguaiacol manganese chloride (EUK-134^{™}).

14. The method as claimed in claim 11, wherein the substance facilitating secretions of extracellular matrices is transforming growth factor beta (TGF-β).
